# EUROPEAN PATENT SPECIFICATION

(11) **EP 2 073 810 B1**
(45) Date of publication and mention of the grant of the patent: **31.08.2011**
(21) Application number: 07842260.7
(22) Date of filing: 11.09.2007
(51) Int. Cl.: A61K 31/513, A61P 3/10, A61P 35/00, A61P 31/18, A61P 5/50

(54) **USE OF 2-6-(3-AMINO-PIPERIDIN-1-YL)-3-METHYL-2,4-DIOXO-3,4-DIHYDRO-2H-PYRIMIDIN-1-YLMETHYL-4-FLUORO-BENZONITRILE FOR TREATING DIABETES, CANCER, AUTOIMMUNE DISORDERS AND HIV INFECTION**
VERWENDUNG VON 2-6-(3-AMIN-PIPERIDIN-1-YL)-3-METHYL-2,4-DIOXO-3,4-DIHYDRO-2H-PYRIMIDIN-1-YLMETHYL-4-FLUOR-BENZONITRIL ZUR BEHANDLUNG VON DIABETES, KREBS, AUTOIMMUNERKRANKUNGEN UND HIV-INFEKTIONEN
ADMINISTRATION DU 2-6-(3-AMINO-PIPERIDIN-1-YL)-3-METHYL-2,4-DIOXO-3,4-DIHYDRO-2H-PYRIMIDIN-1-YLMETHYL-4-FLUORO-BENZONITRILE POUR LE TRAITEMENT DU DIABÈTE, DU CANCER, DES TROUBLES AUTO-IMMUNITAIRES ET DE L'INFECTION PAR LE VIH

(30) Priority: 13.09.2006 WO PCT/US2006/035958; 13.03.2007 US 894628 P
(43) Date of publication of application: 01.07.2009
(73) Proprietor: Takeda Pharmaceutical Company Limited, Osaka- shi, Osaka 541-0045 (JP)
(72) Inventor: CHRISTOPHER, Ronald J., Carlsbad, CA 92011 (US); OGAWA, Atsushi, Osaka 532-8686 (JP); COVINGTON, Paul, Wilmington, North Carolina 28409 (US)
(74) Representative: Duncan, Garreth Andrew
(86) International application number: PCT/US2007/078177
(87) International publication number: WO 2008/033851

(56) References cited:
- EP-A- 1 586 571
- WO-A-2007/033265
- WO-A-2007/033266
- WO-A-2007/033350

## Description

### FIELD OF THE INVENTION

The invention relates to the medical use and dosage of a compound used to inhibit dipeptidyl peptidase IV.

### DESCRIPTION OF RELATED ART

Dipeptidyl Peptidase IV (IUBMB Enzyme Nomenclature EC.3.4.14.5) is a type II membrane protein that has been referred to in the literature by a wide a variety of names including DPP4, DP4, DAP-IV, FAPβ, adenosine deaminase complexing protein 2, adenosine deaminase binding protein (ADAbp), dipeptidyl aminopeptidase IV; Xaa-Prodipeptidyl-aminopeptidase; Gly-Pro naphthylamidase; postproline dipeptidyl aminopeptidase IV; lymphocyte antigen CD26; glycoprotein GP110; dipeptidyl peptidase IV; glycylproline aminopeptidase; glycylproline aminopeptidase; X-prolyl dipeptidyl aminopeptidase; pep X; leukocyte antigen CD26; glycylprolyl dipeptidylaminopeptidase; dipeptidyl-peptide hydrolase; glycylprolyl aminopeptidase; dipeptidyl-aminopeptidase IV; DPP IV/CD26; amino acyl-prolyl dipeptidyl aminopeptidase; T cell triggering molecule Tp103; X-PDAP. Dipeptidyl Peptidase IV is referred to herein as "DPP-IV."

DPP-IV is a non-classical serine aminodipeptidase that removes Xaa-Pro dipeptides from the amino terminus (N-terminus) of polypeptides and proteins. DPP-IV dependent slow release of dipeptides of the type X-Gly or X-Ser has also been reported for some naturally occurring peptides.

DPP-IV is constitutively expressed on epithelial and endothelial cells of a variety of different tissues (intestine, liver, lung, kidney and placenta), and is also found in body fluids. DPP-IV is also expressed on circulating T-lymphocytes and has been shown to be synonymous with the cell-surface antigen, CD-26.

DPP-IV is responsible for the metabolic cleavage of certain endogenous peptides (GLP-1 (7-36), glucagon) *in vivo* and has demonstrated proteolytic activity against a variety of other peptides (GHRH, NPY, GLP-2, VIP) *in vitro.*

GLP-1 (7-36) is a 29 amino-acid peptide derived by post-translational processing of proglucagon in the small intestine. GLP-1 (7-36) has multiple actions *in vivo* including the stimulation of insulin secretion, inhibition of glucagon secretion, the promotion of satiety, and the slowing of gastric emptying. Based on its physiological profile, the actions of GLP-1 (7-36) are believed to be beneficial in the prevention and treatment of type II diabetes and potentially obesity. For example, exogenous administration of GLP-1 (7-36) (continuous infusion) in diabetic patients has been found to be efficacious in this patient population. Unfortunately, GLP-1 (7-36) is degraded rapidly *in vivo* and has been shown to have a short half-life *in vivo* (t_{1/2}=1.5 minutes).

Based on a study of genetically bred DPP-IV knock out mice and on *in vivo* / *in vitro* studies with selective DPP-IV inhibitors, DPP-IV has been shown to be the primary degrading enzyme of GLP-1 (7-36) *in vivo.* GLP-1 (7-36) is degraded by DPP-IV efficiently to GLP-1 (9-36), which has been speculated to act as a physiological antagonist to GLP-1 (7-36). Inhibiting DPP-IV *in vivo* is therefore believed to be useful for potentiating endogenous levels of GLP-1 (7-36) and attenuating the formation of its antagonist GLP-1 (9-36). Thus, DPP-IV inhibitors are believed to be useful agents for the prevention, delay of progression, and/or treatment of conditions mediated by DPP-IV, in particular diabetes and more particularly, type 2 diabetes mellitus, diabetic dislipidemia, conditions of impaired glucose tolerance (IGT), conditions of impaired fasting plasma glucose (IFG), metabolic acidosis, ketosis, appetite regulation and obesity.

Several compounds have been shown to inhibit DPP-IV. Nonetheless, a need still exists for new DPP-IV inhibitors and methods of administering such inhibitors for the treatment of disease.

### SUMMARY OF THE INVENTION

The invention provides compound I for use as a medicament, when administered at a once-per-week dose of between 50 mg and 250 mg of Compound I.

Administering is performed 1 time per week and may optionally be performed 1 time per week as a single dosage. Optionally, administering is performed 1 time per week for a period of at least 30 days and optionally for a period of at least 60 days.

In one variation, administering is performed 1 time per week in the morning and optionally is performed 1 time per week in the morning prior to a first meal of the day for the patient.

Administering may be performed by a wide range of routes of administration including, but not limited to a route selected from the group consisting of orally, parenterally, intraperitoneally, intravenously, intraarterially, transdermally, sublingually, intramuscularly, rectally, transbuccally, intranasally, liposomally, via inhalation, vaginally, intraoccularly, via local delivery, subcutaneously, intraadiposally, intraarticularly, intraperitoneally and intrathecally. In one particular variation, administering is performed orally.

Compound I may be used to treat a range of diseases. In one variation, administering Compound I is performed to treat type I or type II diabetes disease state of the patient. In another variation, administering Compound I is performed to treat a prediabetic patient. In still another variation, administering Compound I is performed to treat an inflammatory bowel disease, Crohn's disease, chemotherapy-induced enteritis, oral mucositis or Shortened Bowel syndrome. In yet another variation, administering Compound I or a pharmaceutical composition comprising Compound I is performed to increase engraftment efficiency after bone marrow transplantation.

In another variation, administering Compound I is performed to treat a patient suffering from conditions mediated by DPP-IV such as diabetes and more particularly, type 2 diabetes mellitus; diabetic dislipidemia; impaired glucose tolerance (IGT); impaired fasting plasma glucose (IFG); metabolic acidosis; ketosis; appetite regulation; obesity; complications associated with diabetes including diabetic neuropathy, diabetic retinopathy and kidney disease; hyperlipidemia including hypertriglyceridemia, hypercholesteremia, hypoHDLemia and postprandial hyperlipidemia; arteriosclerosis; hypertension; myocardial infarction, angina pectoris, cerebral infarction, cerebral apoplexy and metabolic syndrome.

Compound I may be administered in combination with one or more antidiabetic or incretin compounds other than Compound I.

Such combination therapy use is performed where a once-per-week dose of between 50 mg and 250 mg of Compound I, is administered to a patient, In one variation, a once-per-week dose of 50 mg, 75 mg or 100 mg, 150 mg or 200 mg of Compound I is administered to a patient in combination with one or more antidiabetic compounds other than Compound I.

It is noted that several different dosage ranges for particular antidiabetic and incretin compounds are provided herein. It is intended for the scope of the present invention to include drug combinations covering any of the ranges specified in the claims for Compound I in combination with any of the dosage ranges described herein for other antidiabetic or incretin compounds.

Combination of Compound I with one or more antidiabetic compounds other than Compound I provides excellent effects such as 1) enhancement in therapeutic effects of Compound I and/or the antidiabetic compounds; 2) reduction in side effects of Compound I and/or the antidiabetic compounds; and 3) reduction in a dose of Compound I and/or the antidiabetic compounds.

The one or more antidiabetic or incretin compounds administered in combination with Compound I may optionally be selected from the group consisting of insulin signaling pathway modulators, compounds influencing a dysregulated hepatic glucose production, insulin sensitivity enhancers, and insulin secretion enhancers.

The one or more antidiabetic or incretin compounds administered in combination with Compound I may also optionally be selected from the group consisting of protein tyrosine phosphatase inhibitors, glutamine-fructose-6-phosphate amidotransferase inhibitors, glucose-6-phosphatase inhibitors, fructose-1,6-bisphosphatase inhibitors, glycogen phosphorylase inhibitors, glucagon receptor antagonists, phosphoenolpyruvate carboxykinase inhibitors, pyruvate dehydrogenase kinase inhibitors, alpha-glucosidase inhibitors, inhibitors of gastric emptying, glucokinase activators, GLP-1 receptor agonists, GLP-2 receptor agonists, UCP modulators, RXR modulators, GSK-3 inhibitors, PPAR modulators, metformin, insulin, α₂-adrenergic antagonists, deacetylases (*e.g*., reservatrol, sirtuin agonist, polyphenols), and Sodium dependent glucose transport (SGLT2) inhibitors.

The one or more antidiabetic or incretin compounds administered in combination with Compound I may also optionally be selected from the group consisting of GSK-3 inhibitors, retinoid X receptor agonists, Beta-3 AR agonists, UCP modulators, antidiabetic thiazolidinediones, non-glitazone type PPAR gamma agonists, dual PPAR gamma/PPAR alpha agonists, antidiabetic vanadium containing compounds and biguanides.

The one or more antidiabetic or incretin compounds administered in combination with Compound I may also optionally be thiazolidinediones selected from the group consisting of (S)-((3,4-dihydro-2-(phenyl-methyl)-2H-1-benzopyran-6-yl)methyl-thiazolidine-2,4-dione, 5-{[4-(3-(5-methyl-2-phenyl-4-oxazolyl)-1-oxo-propyl)-phenyl]-methyl}-thiazolidine-2,4-dione, 5-{[4-(1-methyl-cyclohexyl)methoxy)-phenyl]methyl]-thiazolidine-2,4-dione, 5-{[4-(2-(1-indolyl)ethoxy)phenyl]methyl}-thiazolidine-2,4-dione, 5-{4-[2-(5-methyl-2-phenyl-4-oxazolyl)-ethoxy)]benzyl}-thiazolidine-2,4-dione, 5-(2-naphthylsulfonyl)-thiazolidine-2,4-dione, bis{4-[(2,4-dioxo-5-thiazolidinyl)-methyl]phenyl} methane, 5-{4-[2-(5-methyl-2-phenyl-4-oxazolyl)-2-hydroxyethoxy]-benzyl}-thiazolidine-2,4-dione, 5-[4-(1-phenyl-1-cyclopropanecarbonylamino)-benzyl]-thiazolidine-2,4-dione, 5-{[4-(2-(2,3-dihydroindol-1-yl)ethoxy)phenylmethyl)-thiazolidine-2,4-dione, 5-[3-(4-chloro-phenyl])-2-propynyl]-5-phenylsulfonyl)thiazolidine-2,4-dione, 5-[3-(4-chlorophenyl])-2-propynyl]-5-(4-fluorophenyl-sulfonyl)thiazolidine-2,4-dione,5-{[4-(2-(methyl-2-pyridinyl-amino)-ethoxy)phenyl]methyl}-thiazolidine-2,4-dione, 5-{[4-(2-(5-ethyl-2-pyridyl)ethoxy)phenyl]-methyl}-thiazolidine-2,4-dione, 5-{[4-((3,4-dihydro-6-hydroxy-2,5,7,8-tetramethyl-2H-1-benzopyran-2-yl)methoxy)-phenyl]-methyl)-thiazolidine-2,4-dione, 5-[6-(2-fluoro-benzyloxy)-naphthalen-2-ylmethyl]-thiazolidine-2,4-dione, 5-([2-(2-naphthyl)-benzoxazol-5-yl]-methyl} thiazolidine-2,4-dione and 5-(2,4-dioxothiazolidin-5-ylmethyl)-2-methoxy-N-(4-trifluoromethyl-benzyl)benzamide, including any pharmaceutically acceptable salts thereof.

In one variation, the one or more antidiabetic compounds administered in combination with Compound I includes metformin. In one particular variation, the metformin in this combination comprises one or more pharmaceutically acceptable salts thereof. In another particular variation, the metformin in this combination comprises a metformin HCl salt. In still another particular variation, the metformin in this combination is administered in a daily dose of between 125 and 2550 mg. In yet another variation, the metformin in this combination is administered in a daily dose of between 250 and 2550 mg. In other variations, the metformin in this combination is administered in an immediate release or extended release formulation.

In another variation, the one or more antidiabetic compounds administered in combination with Compound I includes one or more sulphonyl urea derivatives.

The one or more antidiabetic compounds administered in combination with Compound I may also optionally be selected from the group consisting of glisoxepid, glyburide, glibenclamide, acetohexamide, chloropropamide, glibornuride, tolbutamide, tolazamide, glipizide, carbutamide, gliquidone, glyhexamide, phenbutamide, tolcyclamide, glimepiride and gliclazide, including any pharmaceutically acceptable salts thereof. In one variation, the one or more antidiabetic compounds administered in combination with Compound I includes glimepiride.

The one or more antidiabetic compounds administered in combination with Compound I may also optionally be selected from the group consisting of incretin hormones or mimics thereof, beta-cell imidazoline receptor antagonists, and short-acting insulin secretagogues.

In another variation, the one or more antidiabetic compounds administered in combination with Compound I includes insulin.

The one or more antidiabetic compounds administered in combination with Compound I may also optionally be one or more GLP-1 agonists including, for example, extendatide.

The one or more antidiabetic compounds administered in combination with Compound 1 may also optionally be one or more GLP-2 agonists including, for example, human recombinant GLP-2.

The one or more antidiabetic compounds administered in combination with Compound I may also optionally be one or more antidiabetic D-phenylalanine derivatives.

The one or more antidiabetic compounds administered in combination with Compound I may also optionally be selected from the group consisting of repaglinide, mitiglinide and nateglinide, including any pharmaceutically acceptable salts thereof. In one variation, the one or more antidiabetic compounds administered in combination with Compound I includes mitiglinide calcium salt hydrate.

The one or more antidiabetic compounds administered in combination with Compound I may also optionally be one or more alpha-Glucosidase inhibitors.

The one or more antidiabetic compounds administered in combination with Compound I may also optionally be selected from the group consisting of acarbose, voglibose and miglitol, including any pharmaceutically acceptable salts thereof. In one variation, the one or more antidiabetic compounds administered in combination with Compound I includes voglibose. In another variation, the voglibose in this combination is administered in a daily dose of between 0.1 and 1 mg.

The one or more antidiabetic compounds administered in combination with Compound I may also optionally be rosiglitazone, including any pharmaceutically acceptable salts thereof. In one variation, the rosiglitazone in this combination comprises a rosiglitazone maleate salt

The one or more antidiabetic compounds administered in combination with Compound I may also optionally be tesaglitazar, muraglitazar or naveglitazar, including any pharmaceutically acceptable salts thereof.

The one or more antidiabetic compounds administered in combination with Compound I may also optionally be pioglitazone, including any pharmaceutically acceptable salts thereof. In one variation, the pioglitazone in this combination comprises a pioglitazone HCl salt. In another variation, the pioglitazone in this combination is administered in a daily dose of between 7.5 and 60 mg. In still another variation, the pioglitazone in this combination is administered in a daily dose of between 15 and 45 mg.

The one or more antidiabetic compounds administered in combination with Compound I may also optionally comprise metformin and pioglitazone. In one variation, the pioglitazone in this combination comprises one or more pharmaceutically acceptable salts thereof. In another variation, the pioglitazone in this combination comprises a pioglitazone HCl salt. In still another variation, the pioglitazone in this combination is administered in a daily dose of between 7.5 and 60 mg. In yet another variation, the pioglitazone in this combination is administered in a daily dose of between 15 and 45 mg. In another variation of each of the above variations, the metformin in this combination comprises one or more pharmaceutically acceptable salts thereof. In one particular variation, the metformin in this combination comprises a metformin HCl salt. In another particular variation, the metformin in this combination is administered in a daily dose of between 125 and 2550 mg. In still another variation, the metformin in this combination is administered in a daily dose of between 250 and 2550 mg.

In regard to each of the above embodiments and variations thereof, Compound I may be administered as a free base or as a pharmaceutically acceptable salt thereof. In particular variations, Compound I is administered as a HCl, methanesulfonate, succinate, benzoate, toluenesulfonate, R-(-)mandelate or benzenesulfonate salt of Compound I.

### BRIEF DESCRIPTION OF THE FIGURES

Figure 1 illustrates DPP IV inhibition in plasma after a single oral administration of Compound I in monkey.

Figure 2 illustrates DPP IV inhibition in plasma after a single oral administration of Compound I in human.

Figure 3 illustrates DPP IV inhibition in plasma after a single oral administration of Compound I in human.

### DEFINITIONS

Unless otherwise stated, the following terms used in the specification and claims shall have the following meanings for the purposes of this Application.

"Disease" specifically includes any unhealthy condition of an animal or part thereof and includes an unhealthy condition that may be caused by, or incident to, medical or veterinary therapy applied to that animal, i.e., the "side effects" of such therapy.

"Pharmaceutically acceptable" means that which is useful in preparing a pharmaceutical composition that is generally safe, non-toxic and neither biologically nor otherwise undesirable and includes that which is acceptable for veterinary use as well as human pharmaceutical use.

"Pharmaceutically acceptable salts" means salts which are pharmaceutically acceptable, as defined above, and which possess the desired pharmacological activity. Such salts include, but are not limited to, acid addition salts formed with inorganic acids such as hydrochloric acid, hydrobromic acid, sulfuric acid, nitric acid, phosphoric acid, and the like; or with organic acids such as acetic acid, trifluoroacetic acid, propionic acid, hexanoic acid, heptanoic acid, cyclopentanepropionic acid, glycolic acid, pyruvic acid, lactic acid, malonic acid, succinic acid, malic acid, maleic acid, fumaric acid, tartaric acid, citric acid, benzoic acid, o-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, p-chlorobenzenesulfonic acid, 2-naphthalenesulfonic acid, p-toluenesulfonic acid, camphorsulfonic acid, 4-methylbicyclo[2.2.2]oct-2-ene-1-carboxylic acid, glucoheptonic acid, 4,4'-methylenebis(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid and the like.

Pharmaceutically acceptable salts also include, but are not limited to, base addition salts which may be formed when acidic protons present are capable of reacting with inorganic or organic bases. Acceptable inorganic bases include, but are not limited to, sodium hydroxide, sodium carbonate, potassium hydroxide, aluminum hydroxide and calcium hydroxide. Acceptable organic bases include, but are not limited to, ethanolamine, diethanolamine, triethanolamine, tromethamine, N-methylglucamine and the like.

"Subject" and "patient" includes humans, non-human mammals (e.g., dogs, cats, rabbits, cattle, horses, sheep, goats, swine, deer, and the like) and non-mammals (e.g., birds, and the like).

"Therapeutically effective amount" means that amount of a compound which, when administered to an animal for treating a disease, is sufficient to effect such treatment for the disease.

"Treatment" or "treating" means any administration of a therapeutically effective amount of a compound and includes:
(1) preventing the disease from occurring in an animal which may be predisposed to the disease but does not yet experience or display the pathology or symptomatology of the disease,
(2) inhibiting the disease in an animal that is experiencing or displaying the pathology or symptomatology of the disease (i.e., arresting further development of the pathology and/or symptomatology), or
(3) ameliorating the disease in an animal that is experiencing or displaying the pathology or symptomatology of the disease (i.e., reversing the pathology and/or symptomatology).

### DETAILED DESCRIPTION OF THE INVENTION

### 1. 2-[6-(3-AMINO-PIPERIDIN-1-YL)-3-METHYL-2,4-DIOXO-3,4-DIHYDRO-2H-PYRIMIDIN-1-YLMETHYL]-4-FLUORO-BENZONITRILE AND COMPOSITIONS THEREOF

The present invention relates generally to the administration of 2-[6-(3-Amino-piperidin-1-yl)-3-methyl-2,4-dioxo-3,4-dihydro-2H-pyrimidin-1-ylmethyl]-4-fluoro-benzonitrile (referred to herein as "Compound I") whose structure is provided below.

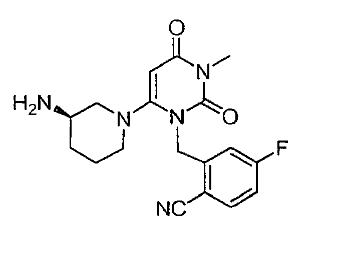

Example 1 describes one method for synthesizing Compound I. It is noted that other methods for synthesizing Compound I may be used as would be appreciated to one of ordinary skill in the art. As described below, Compound I has long acting DPP-IV inhibitory effects.

Compound I may be administered in its free base form and may also be administered in the form of salts and hydrates that are converted *in vivo* into the free base form of Compound I. For example, it is within the scope of the present invention to administer Compound I as a pharmaceutically acceptable salt derived from various organic and inorganic acids and bases in accordance with procedures well known in the art. As used herein, Compound I is intended to encompass salts and hydrates of Compound I unless otherwise specified.

A pharmaceutically acceptable salt of Compound I preferably confers improved pharmacokinetic properties as compared to the free base form Compound I. Pharmaceutically acceptable salts may also initially confer desirable pharmacokinetic properties on Compound I that it did not previously possess, and may even positively affect the pharmacodynamics of the compound with respect to its therapeutic activity in the body.

Particular examples of salts, hydrates and prodrugs of Compound I include, but are not limited to salt forms formed by inorganic or organic acids, e.g., hydrohalides such as hydrochloride, hydrobromide, hydroiodide; other mineral acids and their corresponding salts such as sulfate, nitrate, phosphate, etc.; alkyl and monoarylsulfonates such as ethanesulfonate, toluenesulfonate and benzenesulfonate; and other organic acids and their corresponding salts such as acetate, trifluoroacetate, tartrate, maleate, succinate, citrate, benzoate, salicylate and ascorbate. Further acid addition salts include, but are not limited to: adipate, alginate, arginate, aspartate, bisulfate, bisulfite, bromide, butyrate, camphorate, camphorsulfonate, caprylate, chloride, chlorobenzoate, cyclopentanepropionate, digluconate, dihydrogenphosphate, dinitrobenzoate, dodecylsulfate, fumarate, galacterate (from mucic acid), galacturonate, glucoheptaoate, gluconate, glutamate, glycerophosphate, hemisuccinate, hemisulfate, heptanoate, hexanoate, hippurate, hydrochloride, hydrobromide, hydroiodide, 2-hydroxyethanesulfonate, iodide, isethionate, iso-butyrate, lactate, lactobionate, malate, malonate, mandelate, metaphosphate, methanesulfonate, methylbenzoate, monohydrogenphosphate, 2-naphthalenesulfonate, nicotinate, nitrate, oxalate, oleate, pamoate, pectinate, persulfate, phenylacetate, 3-phenylpropionate, phosphate, phosphonate and phthalate.

In particular variations, Compound I is administered as a HCl, methanesulfonate, succinate, benzoate, toluenesulfonate, R-(-)mandelate or benzenesulfonate salt of Compound I. Example 1 describes the preparation of the succinate salt form of Compound I.

### 2. ADMINISTRATION AND USE OF COMPOUND I

The present invention relates to Compound I for use as a medicament at a once per week dose of between 50 mg/week and 250 mg/week of Compound I, optionally between 50 mg and 200 mg of Compound I, optionally between 50 mg and 150 mg of Compound I, and optionally between 50 mg and 100 mg of Compound I (in each instance based on the molecular weight of the free base form of Compound I). Specific dosage amounts that may be used include, but are not limited to 50 mg, 75 mg and 100 mg of Compound I per week on a once-per-week regimen. It is noted that unless otherwise specifically specified, Compound I may be administered in its free base form or as a pharmaceutically acceptable salt. However, the dosage amounts and ranges provided herein are always based on the molecular weight of the free base form of Compound I.

Compound I may be administered by any route of administration. In particular embodiments, however, the method of the present invention is practiced by administering Compound I orally. This type of administration is advantageous in that it is easy and may be self-administered by the patient.

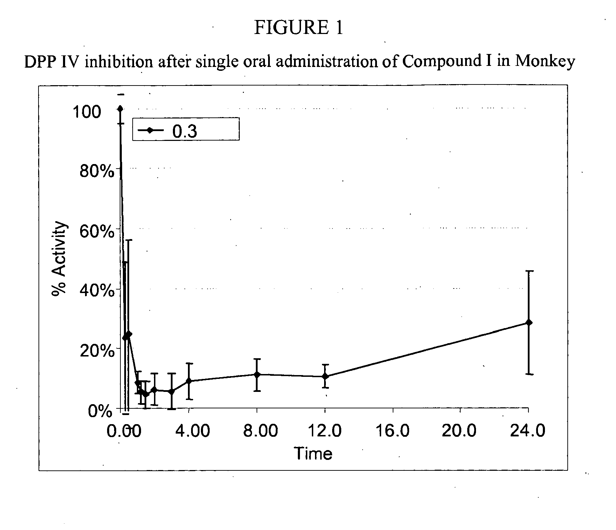

Compound I may be administered to any patient who would benefit from a course of treatment leading to the reduction of in vivo DPP-IV activity. Figure 1 illustrates (for reference purposes) and Example 3 describes the observed effect that administering Compound I has on monkey plasma DPPIV activity after a single oral administration. As can be seen from the data shown in Figure 1, by administering Compound I one time per day at the dosage levels specified herein, Compound I can be effectively used relative to disease states where it is desired to reduce plasma DPPIV activity. In view of the data presented, it is believed that when at least 2.5 mg of Compound I is administered to a patient, the patient's plasma DPPIV activity may be reduced by greater than 60% relative to baseline for a period of at least at least 6 hours, 12 hours, 18 hours and even 24 hours following administration.

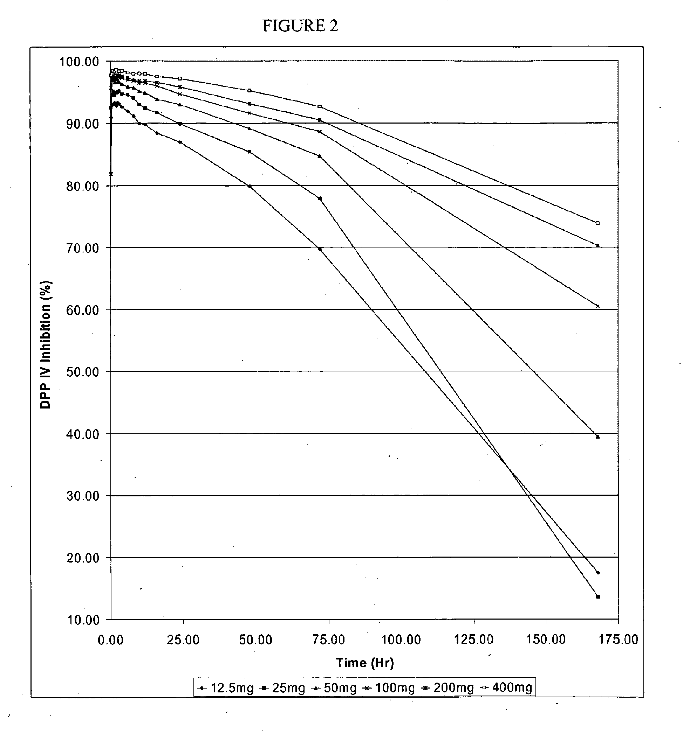

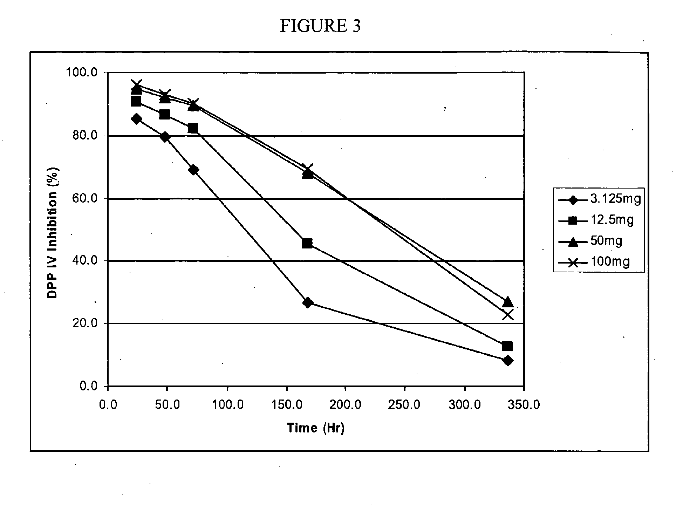

In addition, as described in detail below, Figures 2 and 3 illustrate the observed effect that administering Compound I has on human plasma DPP-IV activity after a single oral administration. As can be seen from the data shown in Figure 2, by administering Compound 1 at the dosage levels specified herein, Compound I can be effectively used relative to disease states where it is desired to reduce plasma DPP-IV activity. In view of the data presented, it is believed that when at least 12.5 mg of Compound I is administered to a patient, the patient's plasma DPP-IV activity may be reduced by greater than 10% relative to baseline for a period of at least 168 hours following administration; when at least 50 mg of Compound I is administered to a patient, the patient's plasma DPP-IV activity may be reduced by greater than 35% relative to baseline for a period of at least 168 hours following administration; and when 200 mg or 400 mg of Compound I is administered to a patient, the patient's plasma DPP-IV activity may be reduced by greater than 70% relative to baseline for a period of at least 168 hours following administration.Examples of particular applications for administering Compound I include, but are not limited to the prevention, delay of progression, and/or treatment of conditions mediated by DPP-IV, in particular diabetes and more particularly, type 2 diabetes mellitus, diabetic dislipidemia, impaired glucose tolerance (IGT), impaired fasting plasma glucose (IFG), metabolic acidosis, ketosis, appetite regulation, obesity and complications associated with diabetes including diabetic neuropathy, diabetic retinopathy, inflammatory bowel disease, Crohn's disease, chemotherapy-induced enteritis, oral mucositis, Shorthened Bowel Syndrome and kidney disease. The conditions mediated by DPP-IV further includes hyperlipidemia such as hypertriglyceridemia, hypercholesteremia, hypoHDLemia and postprandial hyperlipidemia; arteriosclerosis; hypertension; myocardial infarction, angina pectoris, cerebral infarction, cerebral apoplexy and metabolic syndrome.

It is believed that administration of Compound I to type I or type II diabetic patients following a minimum treatment of at least 30 days will improve one or more cardiovascular measurements. Examples of cardiac measurements that may be improved include, but are not limited to a decrease in mean systolic blood pressure, an increase in HDL cholesterol, improvement in LDL/HDL ratio and a reduction in triglycerides.

It is also believed that administration of Compound I in combination with one or more antidiabetic compounds to type I or type II diabetic patients following a minimum treatment of at least 30 days will improve one or more cardiovascular measurements. Examples of cardiac measurements that may be improved include, but are not limited to a decrease in mean systolic blood pressure, an increase in HDL cholesterol, improvement in LDL/HDL ratio and a reduction in triglycerides.

It is also believed that administration of Compound I in combination with one or more antidiabetic or incretin compounds to patients with gastrointestinal inflammatory disorders (including, but not be limited to inflammatory bowel disease, Crohn's disease, chemotherapy-induced enteritis, oral mucositis and Shortened Bowel Syndrome) following a minimum treatment of at least 30 days will improve the health of the mucosal lining of the gastrointestinal tract. Improvement in the health of the mucosal lining of the gastrointestinal tract may be demonstrated by, but is not limited to, an increase in the intestinal surface area, reduced inflammation, and/or increases in absorption of nutrients.

In one variation, Compound I is administered to a patient with type 2 diabetes. Patients receiving Compound I may also have a malfunction in insulin secretion from pancreatic islets rather than patients who have developed insulin resistance in peripheral insulin sensitive tissues/organs.

Advantageously, administering Compound I one time per week, at the dosage levels specified herein may also be used to treat patients who are prediabetic. It is believed that administering Compound I in a patient who is prediabetic serves to delay development of type II diabetes in that patient. Sustained increase in blood glucose desensitizes pancreatic islet function and impairs insulin secretion. By improving cyclic AMP levels and the calcium dynamics in beta cells, the cells activate genes repairing damaged cell components and are less vulnerable to glucose toxicity.

Administering Compound I one time per week at the dosage levels specified herein is expected to have a range of desirous biological effects *in vivo.* For example, administering Compound I one time per week, at the dosage levels specified herein reduces the patient's blood glucose level when compared with placebo control. Such a decrease in postprandial blood glucose levels helps diabetic patients to maintain lower glucose levels.

Administering Compound I one time per week, at the dosage levels specified herein is also expected to have the effect of increasing the patient's insulin level or insulin sensitivity. Insulin facilitates entry of glucose into muscle, adipose and several other tissues. The mechanism by which cells can take up glucose is by facilitated diffusion through stimulation of insulin receptor. C-peptide and insulin are protein chains created by the activation and division of proinsulin (an inactive precursor to insulin). C-peptide and insulin are created and stored in the beta cells of the pancreas. When insulin is released into the bloodstream, equal amounts of C-peptide also are released. This makes C-peptide useful as a marker of insulin production. Administering Compound I according to the present invention is expected to increase the patient's C-peptide level.

Administering Compound I one time per week at the dosage levels specified herein is also expected to have the affect of decreasing the patient's hemoglobin Alc level by greater than 0.2% when compared to placebo control after extended treatment with Compound I. Hb-A1c values are known to be directly proportional to the concentration of glucose in the blood over the life span of the red blood cells. Hb-A1c c thus gives an indication of a patient's blood glucose levels over the previous last 90 days, skewed to the most recent 30 days.

### 3. COMBINATION THERAPY INCLUDING COMPOUND I

The present invention also relates to the use of Compound I in combination with one or more other antidiabetic and/or incretin compounds. Examples of such other antidiabetic compounds include, but are not limited to insulin signaling pathway modulators, like protein tyrosine phosphatase (PTPase) inhibitors, and glutamine-fructose-6-phosphate amidotransferase (GFAT) inhibitors; compounds influencing a dysregulated hepatic glucose production, like glucose-6-phosphatase (G6Pase) inhibitors, fructose-1,6-bisphosphatase (F-1,6-BPase) inhibitors, glycogen phosphorylase (GP) inhibitors, glucagon receptor antagonists and phosphoenolpyruvate carboxykinase (PEPCK) inhibitors; pyruvate dehydrogenase kinase (PDHK) inhibitors; insulin sensitivity enhancers (insulin sensitizers); insulin secretion enhancers (insulin secretagogues); alpha-glucosidase inhibitors; inhibitors of gastric emptying; glucokinase activators, GLP-1 receptor agonists, GLP-2 receptor agonists, UCP modulators, RXR modulators, GSK-3 inhibitors, PPAR modulators, metformin, insulin; and α₂-adrenergic antagonists. Compound I may be administered with such at least one other antidiabetic compound either simultaneously as a single dose, at the same time as separate doses, or sequentially (i.e., where one is administered before or after the other is administered).

Examples of PTPase inhibitors that may be used in combination with Compound I include, but are not limited to those disclosed in U.S. Patent. Nos. 6,057,316, 6,001,867, and PCT Publication Nos. WO 99/58518, WO 99/58522, WO 99/46268, WO 99/46267, WO 99/46244, WO 99/46237, WO 99/46236, and WO 99/15529.

Examples of GFAT inhibitors that may be used in combination with Compound I include, but are not limited to those disclosed in Mol. Cell. Endocrinol. 1997, 135(1),67-77.

Examples of G6Pase inhibitors that may be used in combination with Compound I include, but are not limited to those disclosed in PCT Publication Nos. WO 00/14090, WO 99/40062 and WO 98/40385, European Patent Publication No. EP682024 and Diabetes 1998, 47,1630-1636.

Examples of F-1,6-BPase inhibitors that may be used in combination with Compound I include, but are not limited to those disclosed in PCT Publication Nos. WO 00/14095, WO 99/47549, WO 98/39344, WO 98/39343 and WO 98/39342.

Examples of GP inhibitors that may be used in combination with Compound I include, but are not limited to those disclosed in U.S. Patent No. 5,998,463, PCT Publication Nos. WO 99/26659, WO 97/31901, WO 96/39384 and WO9639385 and European Patent Publication Nos. EP 978279 and EP 846464.

Examples of glucagon receptor antagonists that may be used in combination with Compound I include, but are not limited to those disclosed in U.S. Patent Nos. 5,880,139 and 5,776,954, PCT Publication Nos. WO 99/01423, WO 98/22109, WO 98/22108, WO 98/21957, WO 97/16442 and WO 98/04528 and those described in Bioorg Med. Chem. Lett 1992, 2, 915-918, J. Med. Chem. 1998, 41, 5150-5157, and J. Biol Chem. 1999, 274; 8694-8697.

Examples of PEPCK inhibitors that may be used in combination with Compound I include, but are not limited to those disclosed in U.S. Patent No. 6,030,837 and Mol. Biol. Diabetes 1994,2, 283-99.

Examples of PDHK inhibitors that may be used in combination with Compound I include, but are not limited to those disclosed in J. Med. Chem. 42 (1999) 2741-2746.

Examples of insulin sensitivity enhancers that may be used in combination with Compound I include, but are not limited to GSK-3 inhibitors, retinoid X receptor (RXR) agonists, Beta-3 AR agonists, UCP modulators, antidiabetic thiazolidinediones (glitazones), non-glitazone type PPAR gamma agonists, dual PPAR gamma/PPAR alpha agonists, antidiabetic vanadium containing compounds and biguanides such as metformin.

Examples of GSK-3 inhibitors include, but are not limited to those disclosed in PCT Publication Nos. WO 00/21927 and WO 97/41854.

Examples of RXR modulators include, but are not limited to those disclosed in U.S. Patent Nos. 4,981,784, 5,071,773, 5,298,429 and 5,506,102 and PCT Publication Nos. WO89/05355, WO91/06677, WO92/05447, WO93/11235, WO95/18380, WO94/2306, and WO93/23431.

Examples of Beta-3 AR agonists include, but are not limited to CL-316,243 (Lederle Laboratories) and those disclosed in U.S. Patent No. 5,705,515 and PCT Publication Nos. WO 99/29672, WO 98/32753, WO 98/20005, WO 98/09625, WO 97/46556, and WO 97/37646.

Examples of UCP modulators include agonists of UCP-1, UCP-2 and UCP-3. Examples of UCP modulators include, but are not limited to those disclosed in Vidal-Puig et al., Biochem. Biophys. Res. Commun., Vol. 235(1) pp. 79-82 (1997).

Examples of antidiabetic, PPAR modulating thiazolidinediones (glitazones) include, but are not limited to, (S)-((3,4-dihydro-2-(phenyl-methyl)-2H-1-benzopyran-6-yl)methyl-thiazolidine-2,4-dione (englitazone), 5-{[4-(3-(5-methyl-2-phenyl-4-oxazolyl)-1-oxo-propyl)-phenyl]-methyl}-thiazolidine-2,4-dione (darglitazone), 5-{[4-(1-methylcyclohexyl)methoxy)-phenyl]methyl]-thiazolidine-2,4-dione (ciglitazone), 5-{[4-(2-(1-indolyl)ethoxy)phenyl]methyl}-thiazolidine-2,4-dione (DRF2189), 5-{4-[2-(5-methyl-2-phenyl-4-oxazoly)-ethoxy)]benzyl}-thiazolidine-2,4-dione (BM-13.1246), 5-(2-naphthylsulfonyl)-thiazolidine-2,4-dione (AY-31637), bis{4-[(2,4-dioxo-5-thiazolidinyl)-methyl]phenyl} methane (YM268), 5- {4-[2-(5-methyl-2-phenyl-4-oxazolyl)-2-hydroxyethoxy]-benzyl}- -thiazolidine-2,4-dione (AD-5075), 5-[4-(1-phenyl-1-cyclopropanecarbonylamino)-benzyl]-thiazolidine-2,4-dione (DN-108) 5-{[4-(2-(2,3-dihydroindol-1-yl)ethoxy)phenylmethyl)-thiazolidine-2,4-dione, 5-[3-(4-chloro-phenyl])-2-- propynyl]-5-phenylsulfonyl)thiazolidine-2,4-dione, 5-[3-(4-chlorophenyl])-- 2-propynyl]-5-(4-fluorophenyl-sulfonyl)thiazolidine-2,4-dione,5-{[4-(2-(methyl-2-pyridinyl-amino)-ethoxy)phenyl]methyl}-thiazolidine-2,4-dione (rosiglitazone), 5-{[4-(2-(5-ethyl-2-pyridyl)ethoxy)phenyl]-methyl }-thiazolidine-2,4-dione (pioglitazone; marketed under the trademark ACTOS™), 5-[6-(2-fluoro-benzyloxy)-naphthalen-2-ylmethyl]-thiazolidine-2,4-dione (MCC555), 5-([2-(2-naphthyl)-benzoxazol-5-yl]-methyl}thiazolidine-2,4-dione (T-174), edaglitazone (BM-13-1258), rivoglitazone (CS-011), and 5-(2,4-dioxothiazolidin-5-ylmethyl)-2-methoxy-N-(4-trifluoromethylbenzyl)benzamide (KRP297).

Examples of non-glitazone type PPAR gamma agonists include, but are not limited to N-(2-benzoylphenyl)-L-tyrosine analogues, such as GI-262570, reglixane (JTT501) and FK-614 and metaglidasen (MBX-102).

Examples of dual PPAR gamma/PPAR alpha agonists include, but are not limited to omega.-[(oxoquinazolinylalkoxy)phenyl]alkanoates and analogs thereof including those described in PCT Publication No. WO 99/08501 and Diabetes 2000, 49(5), 759-767; tesaglitazar, muraglitazar and naveglitazar.

Examples of antidiabetic vanadium containing compounds include, but are not limited to those disclosed in the U.S. Patent No. 5,866,563.

Metformin (dimethyldiguanide) and its hydrochloride salt is marketed under the trademark GLUCOPHAGE™.

Examples of insulin secretion enhancers include but are not limited to glucagon receptor antagonists (as described above), sulphonyl urea derivatives, incretin hormones or mimics thereof, especially glucagon-like peptide-1 (GLP-1) or GLP-1 agonists, beta-cell imidazoline receptor antagonists, and short-acting insulin secretagogues, like antidiabetic phenylacetic acid derivatives, antidiabetic D-phenylalanine derivatives, and mitiglinide and pharmaceutical acceptable salts thereof.

Examples of sulphonyl urea derivatives include, but are not limited to, glisoxepid, glyburide, glibenclamide, acetohexamide, chloropropamide, glibornuride, tolbutamide, tolazamide, glipizide, carbutamide, gliquidone, glyhexamide, phenbutamide, tolcyclamide; glimepiride and gliclazide. Tolbutamide, glibenclamide, gliclazide, glibornuride, gliquidone, glisoxepid and glimepiride can be administered in the form that they are marketed under the trademarks RASTINON HOECHST™, AZUGLUCON™, DIAMICRONT™, GLUBORID™, GLURENORM™, PRO-DIABAN™ and AMARYL™, respectively.

Examples of GLP-1 agonists include, but are not limited to those disclosed in U.S. Patent Nos. 5,120,712, 5,118,666 and 5,512,549, and PCT Publication No. WO 91/11457. In particular, GLP-1 agonists include those compounds like GLP-1 (7-37) in which compound the carboxy-terminal amide functionality of Arg³⁶ is displaced with Gly at the 37^{th} position of the GLP-1 (7-36)NH₂ molecule and variants and analogs thereof including GLN⁹-GLP-1 (7-37), D-GLN⁹-GLP-1 (7-37), acetyl LYS⁹-GLP-1 (7-37), LYS¹⁸-GLP-1 (7-37) and, in particular, GLP-1 (7-37)OH, VAL⁸-GLP-1 (7-37), GLY⁸-GLP-1(7-37), THR⁸-GLP-1 (7-37), GLP-1 (7-37) and 4-imidazopropionyl-GLP-1.

One particular example of a GLP-1 agonist is Extendatide, a 39-amino acid peptide amide, which is marketed under the trademark BYETTA™. Exenatide has the empirical formula C₁₈₄H₂₈₂N₅₀O₆₀S and molecular weight of 4186.6 Daltons. The amino acid sequence for Exenatide is as follows: H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH₂

Examples of glucagon-like peptide-2 (GLP-2) or GLP-2 agonists include, but are not limited to those disclosed in U.S. Patent No. 7,056,886 and PCT Publication Nos. WO 00/53208, WO 01/49314 and WO 03/099854. One particular example of a GLP-2 agonist is TEDUGLUTIDE™, a 39-amino acid peptide amide (NPS Pharmaceuticals, Inc.).

Examples of beta-cell imidazoline receptor antagonists include, but are not limited to those described in PCT Publication No. WO 00/78726 and J. Pharmacol. Exp. Ther. 1996; 278; 82-89.

An example of an antidiabetic phenylacetic acid derivative is repaglinide and pharmaceutically acceptable salts thereof.

Examples of antidiabetic D-phenylalanine derivatives include, but are not limited to nateglinide (N-[(trans4-isopropylcyclohexyl)-carbonyl]-D-phenylalanine, EP 196222 and EP 526171) and repaglinide ((S)-2-ethoxy-4-{2-[[3-methy-1-1-[2-(1-piperidinyl)phenyl]butyl]-amino]-2-oxoethyl}benzoic acid, EP 0 147 850 A2 and EP 0 207 331 A1). Nateglinide is intended to include the particular crystal forms (polymorphs) disclosed in U.S. Patent No. 5,488,510 and European Patent Publication No. EP 0526171 B1. Repaglinide and nateglinide may be administered in the form as they are marketed under the trademarks NOVONORM™ and STARLIX™, respectively.

Examples of alpha-Glucosidase inhibitors include, but are not limited to, acarbose, N-(1,3-dihydroxy-2-propyl)valiolamine (voglibose) and the 1-deoxynojirimycin derivative miglitol. Acarbose is 4",6"-dideoxy-4'-[(1S)-(1,4,6/5)-4,5,6-trihydroxy-3-hydroxymethyl-2-cyclo-hexenylamino)maltotriose. The structure of acarbose can as well be described as O-4,6-dideoxy-4-{[1S,4R,5S,6S]-4,5,6-trihydroxy-3-(hydroxymethyl)-2-cyclohexen-1-yl]-amino)-alpha-D-glucopyranosyl-(1-4)-O- alpha-D-glucopyranosyl-(1-4)-D-glucopyranose. (U.S. Patent No. 4,062,950 and European Patent Publication No. EP 0 226 121). Acarbose and miglitol may be administered in the forms that they are marketed under the trademarks GLUCOBAY™ and DIASTABOL 50™ respectively.

Examples of inhibitors of gastric emptying other than GLP-1 include, but are not limited to those disclosed in J. Clin. Endocrinol. Metab. 2000, 85(3), 1043-1048, and Diabetes Care 1998; 21; 897-893, especially Amylin and analogs thereof such as pramlintide. Amylin is described in Diabetologia 39, 1996, 492-499.

Examples of α₂-adrenergic antagonists include, but are not limited to midaglizole which is described in Diabetes 36,1987, 216-220. The insulin that may be used in combination with Compound I include, but are not limited to animal insulin preparations extracted from the pancreas of bovine and pig; human insulin preparations genetically synthesized using *Escherichia coli* or yeast; zinc insulin; protamine zinc insulin; fragment or derivative of insulin (e.g., INS-1) and an oral insulin preparation.

In one particular embodiment, the antidiabetic compound administered in combination with Compound I is selected from the group consisting of nateglinide, mitiglinide, repaglinide, metformin, extendatide, rosiglitazone, tesaglitazar, pioglitazone, glisoxepid, glyburide, glibenclamide, acetohexamide, chloropropamide, glibornuride, tolbutamide, tolazamide, glipizide, carbutamide, gliquidone, glyhexamide, phenbutamide, tolcyclamide, glimepiride and gliclazide, including any pharmaceutically acceptable salts thereof.

Examples of the preparation and formulation of PTPase inhibitors, GSK-3 inhibitors, non-small molecule mimetic compounds, GFAT inhibitors, G6Pase inhibitors, glucagon receptor antagonists, PEPCK inhibitors, F-1,6-BPase inhibitors, GP inhibitors, RXR modulators, Beta-3 AR agonists, PDHK inhibitors, inhibitors of gastric emptying and UCP modulators are disclosed in the patents, applications and references provided herein.

In the case of combination therapy with Compound I, the other antidiabetic compound may be administered (e.g., route and dosage form) in a manner known per se for such compound. Compound I and the other antidiabetic compound may be administered sequentially (i.e., at separate times) or at the same time, either one after the other separately in two separate dose forms or in one combined, single dose form. In one particular embodiment, the other antidiabetic compound is administered with Compound I as a single, combined dosage form. The dose of the antidiabetic compound may be selected from the range known to be clinically employed for such compound. Any of therapeutic compounds of diabetic complications, antihyperlipemic compounds, antiobestic compounds or antihypertensive compounds can be used in combination with Compound I in the same manner as the above antidiabetic compounds. Examples of therapeutic compounds of diabetic complications include, but are not limited to, aldose reductase inhibitors such as tolrestat, epalrestat, zenarestat, zopolrestat, minalrestat, fidarestat, CT-112 and ranirestat; neurotrophic factors and increasing compounds thereof such as NGF, NT-3, BDNF and neurotrophin production-secretion promoters described in WO01/14372 (e.g., 4-(4-chlorophenyl)-2-(2-methyl-1-imidazolyl)-5-[3-(2-methylphenoxy)propyl]oxazole); neuranagenesis stimulators such as Y-128; PKC inhibitors such as ruboxistaurin mesylate; AGE inhibitors such as ALT946, pimagedine, N-phenacylthiazolium bromide (ALT766), ALT-711, EXO-226, pyridorin and pyridoxamine; reactive oxygen scavengers such as thioctic acid; cerebral vasodilators such as tiapride and mexiletine; somatostatin receptor agonists such as BIM23190; and apoptosis signal regulating kinase-1 (ASK-1) inhibitors. Examples of antihyperlipemic compounds include, but are not limited to, HMG-CoA reductase inhibitors such as pravastatin, simvastatin, lovastatin, atorvastatin, fluvastatin, rosuvastatin and pitavastatin; squalene synthase inhibitors such as compounds described in WO97/10224 (e.g., N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid); fibrate compounds such as bezafibrate, clofibrate, simfibrate and clinofibrate; ACAT inhibitors such as avasimibe, and eflucimibe; anion exchange resins such as colestyramine; probucol; nicotinic acid drugs such as nicomol and niceritrol; ethyl icosapentate; and plant sterols such as soysterol and γ-oryzanol. Examples of antiobestic compounds include, but are not limited to, dexfenfluramine, fenfluramine, phentermine, sibutramine, amfepramone, dexamphetamine, mazindol, phenylpropanolamine, clobenzorex; MCH receptor antagonists such as SB-568849 and SNAP-7941; neuropeptide Y antagonists such as CP-422935; cannabinoid receptor antagonists such as SR-141716 and SR-147778; ghrelin antagonist; 11 β-hydroxysteroid dehydrogenase inhibitors such as BVT-3498; pancreatic lipase inhibitors such as orlistat and ATL-962; Beta-3 AR agonists such as AJ-9677; peptidic anorexiants such as leptin and CNTF (Ciliary Neurotropic Factor); cholecystokinin agonists such as lintitript and FPL-15849; and feeding deterrent such as P-57. Examples of the antihypertensive compounds include angiotensin converting enzyme inhibitors such as captopril, enalapril and delapril; angiotensin II antagonists such as candesartan cilexetil, losartan, eprosartan, valsartan, telmisartan, irbesartan, olmesartan medoxomil, tasosartan and 1-[[2'-(2,5-dihydro-5-oxo-4H-1,2,4-oxadiazol-3-yl)biphenyl-4-yl]methyl]-2-ethoxy-1H-benzimidazole-7-carboxylic acid; calcium channel blockers such as manidipine, nifedipine, nicardipine, amlodipine and efonidipine; potassium channel openers such as levcromakalim, L-27152, AL0671 and NIP-121; clonidine; deacetylases such as reservatrol, sirtuin agonist, polyphenols; MCR4 agonist; Sodium dependent glucose transport (SGLT2) inhibitors.

The structure of the active agents identified herein by code nos., generic or trade names may be taken from the actual edition of the standard compendium "The Merck Index" or from databases, e.g. Patents International (e.g. IMS World Publications). The corresponding content thereof is hereby incorporated by reference. Any person skilled in the art is fully enabled to identify the active agents and, based on these references, likewise enabled to manufacture and test the pharmaceutical indications and properties in standard test models, both in vitro and in vivo.

### 4. COMPOSITIONS COMPRISING COMPOUND I

Compound I may be comprised within a pharmaceutical composition adapted for a variety of routes of administration. For example, Compound I may be comprised within a pharmaceutical composition adapted to be administered by a route selected from the group consisting of orally, parenterally, intraperitoneally, intravenously, intraarterially, transdermally, sublingually, intramuscularly, rectally, transbuccally, intranasally, liposomally, via inhalation, vaginally, intraoccularly, via local delivery (for example by catheter or stent), subcutaneously, intraadiposally, intraarticularly, intraperitoneally and intrathecally. As such, Compound I may be formulated in a variety of pharmaceutically acceptable compositions including injectable forms (e.g. subcutaneous, intravenous, intramuscular and intraperitoneal injections), drip infusions, external application forms (e.g. nasal spray preparations, transdermal preparations; ointments, etc.), and suppositories (e.g. rectal and vaginal suppositories). These different pharmaceutically acceptable compositions can be manufactured by known techniques conventionally used in the pharmaceutical industry with a pharmaceutically acceptable carrier conventionally used in the pharmaceutical industry.

As used herein, a composition comprising Compound I is intended to encompass the free base form of Compound I, salts and hydrates of Compound I, as well as other materials that may be included in such composition for its intended purpose, including other active ingredients, unless otherwise specified. Particular salt forms of Compound I that may be employed include, but are not limited to, the HCl, methanesulfonate, succinate, benzoate, toluenesulfonate, R-(-)mandelate or benzenesulfonate salt forms of Compound I. As noted above, Compound I may advantageously be used when administered to a patient at a daily dose of between 50 mg/week and 250 mg/week of Compound 1 to a patient, optionally between 50 mg and 200 mg of Compound I, optionally between 50 mg and 150 mg of Compound I, and optionally between 50 mg and 100 mg of Compound I (in each instance based on the molecular weight of the free base form of Compound I). Specific dosage amounts that may be used include, but are not limited to 50 mg, 75 mg and 100 mg of Compound I per week.

As also noted above, Compound I may advantageously be used when administered orally. Accordingly, the compositions of the present invention may optionally be adapted for oral administration. In one variation, such pharmaceutical composition is a solid formulation adapted for oral administration. In this regard, the composition, for example, may be in the form of a tablet or capsule. Example 2 provides examples of solid formulations comprising Compound I adapted for oral administration. In another variation, such pharmaceutical composition is a liquid formulation adapted for oral administration.

As also noted above, Compound I may advantageously be used when administered parenterally. Accordingly, the compositions of the present invention may optionally be adapted for parenteral administration. In one variation, such pharmaceutical composition is a solution formulation adapted for parenteral administration. In another variation, such pharmaceutical composition is a suspension formulation adapted for parenteral administration.

As noted above, Compound I may advantageously be used in combination with one or more other antidiabetic and/or incretin compounds. Accordingly, the compositions of the present invention may optionally comprises Compound I in combination with one or more other antidiabetic or incretin compounds in a combined, single dose form.

Optionally, such combined, single dose form comprising Compound I in combination with one or more other antidiabetic and/or incretin compounds is adapted for oral administration and optionally is a solid oral dose form. Alternatively, such combined, single dose form comprising Compound I in combination with one or more other antidiabetic and/or incretin compounds can be adapted for parenteral administration and optionally is a solution dose form.

In one variation, such combined, single dose form comprising Compound I in combination with one or more other antidiabetic compounds comprises between 50 mg/week and 250 mg/week of Compound I to a patient, optionally between 50 mg and 200 mg of Compound I, optionally between 50 mg and 150 mg of Compound I, and optionally between 50 mg and 100 mg of Compound I (in each instance based on the molecular weight of the free base form of Compound I). In specific embodiments, such combined, single dose form comprising Compound I in combination with one or more other antidiabetic compounds comprises 50 mg, 75 mg, and 100 mg of Compound I.

Any antidiabetic compound, or set of antidiabetic compounds may be combined with Compound I to form such combined, single dose form. In particular embodiments, such combined, single dose form includes Compound I and one or more members of the group consisting of insulin signaling pathway modulators, like protein tyrosine phosphatase (PTPase) inhibitors, and glutamine-fructose-6-phosphate amidotransferase (GFAT) inhibitors, compounds influencing a dysregulated hepatic glucose production, like glucose-6-phosphatase (G6Pase) inhibitors, fructose-1,6-bisphosphatase (F-1,6-BPase) inhibitors, glycogen phosphorylase (GP) inhibitors, glucagon receptor antagonists and phosphoenolpyruvate carboxykinase (PEPCK) inhibitors, pyruvate dehydrogenase kinase (PDHK) inhibitors, insulin sensitivity enhancers (insulin sensitizers), insulin secretion enhancers (insulin secretagogues), alpha-glucosidase inhibitors, inhibitors of gastric emptying, glucokinase activators, GLP-1 receptor agonists, GLP-2 receptor agonists, UCP modulators, RXR modulators, GSK-3 inhibitors, PPAR modulators, metformin, insulin, and α₂-adrenergic antagonists. Compound I may be administered with such at least one other antidiabetic compound either simultaneously as a single dose, at the same time as separate doses, or sequentially (i.e., where on is administered before or after the other is administered).

In one variation, such combined, single dose form comprises Compound I and an antidiabetic thiazolidinedione. Particular examples of thiazolidinediones that may be used in this variation include, but are not limited to (S)-((3,4-dihydro-2-(phenyl-methyl)-2H-1-benzopyran-6-yl)methyl-thiazolidine-2,4-dione (englitazone), 5-{[4-(3-(5-methyl-2-phenyl-4-oxazolyl)-1-oxo-propyl)-phenyl]-methyl}-thiazolidine-2,4-dione (darglitazone), 5-{[4-(1-methyl-cyclohexyl)methoxy)-phenyl]methyl]-thiazolidine-2,4-dione (ciglitazone), 5-{[4-(2-(1-indolyl)ethoxy)phenyl]methyl}-thiazolidine-2,4-dione (DRF2189), 5-{4-[2-(5-methyl-2-phenyl-4-oxazoly)-ethoxy)]benzyl}-thiazolidine-2,4-dione (BM-13.1246), 5-(2-naphthylsulfonyl)-thiazolidine-2,4-dione (AY-31637), bis{4-[(2,4-dioxo-5-thiazolidinyl)-methyl]phenyl}methan- e (YM268), 5-{4-[2-(5-methyl-2-phenyl-4-oxazolyl)-2-hydroxyethoxy]-benzyl} -thiazolidine-2,4-dione (AD-5075), 5-[4-(1-phenyl-1-cyclopropanecarbonylamino)-benzyl]-thiazolidine-2,4-dione (DN-108) 5-{[4-(2-(2,3-dihydroindol-1-yl)ethoxy)phenylmethyl)-thiazolidine-2,4-dione, 5-[3-(4-chlorophenyl])-2-- propynyl]-5-phenylsulfonyl)thiazolidine-2,4-dione, 5-[3-(4-chlorophenyl])-2-propynyl]-5-(4-fluorophenyl-sulfonyl)thiazolidine-2,4-dione, 5-{[4-(2-(methyl-2-pyridinyl-amino)-ethoxy)phenyl]methyl}-thiazolidine-2,4-dione (rosiglitazone), 5-{[4-(2-(5-ethyl-2-pyridyl)ethoxy)phenyl]-methyl-thiazolidine-2,4-dione (pioglitazone), 5-[6-(2-fluoro-benzyloxy)-naphthalen-2-ylmethyl]-thiazolidine-2,4-dione (MCC555), 5-([2-(2-naphthyl)-benzoxazol-5-yl]-methyl}thiazolidine-2,4-dione (T-174), edaglitazone (BM-13-1258), rivoglitazone (CS-011) and 5-(2,4-dioxothiazolidin-5-ylmethyl)-2-met- hoxy-N-(4-trifluoromethyl-benzyl)benzamide (KRP297).

In one particular variation, the thiazolidinedione in such combined, single dose form is 5-{[4-(2-(5-ethyl-2-pyridyl)ethoxy)phenyl]-methyl}-thiazolidine-2,4-dione (pioglitazone) and its hydrochloride salt which is marketed under the trademark ACTOS™.

In another particular variation, the thiazolidinedione is 5-{[4-(2-(methyl-2-pyridinyl-amino)-ethoxy)phenyl]methyl}-thiazolidine-2,4-dione (rosiglitazone) and its maleate salt.

In another variation, such combined, single dose form comprises Compound I and a non-glitazone type PPAR gamma agonist.

In another variation, such combined, single dose form comprises Compound I and a biguanide. A particular example of a biguanide that may be used in this variation is Metformin (dimethyldiguanide) and its hydrochloride salt which is marketed under the trademark GLUCOPHAGE™.

In another variation, such combined, single dose form comprises Compound I and a sulphonyl urea derivative. Particular examples of sulphonyl urea derivatives that may be used in this variation include, but are not limited to glisoxepid, glyburide, glibenclamide, acetohexamide, chloropropamide, glibornuride, tolbutamide, tolazamide, glipizide, carbutamide, gliquidone, glyhexamide, phenbutamide, tolcyclamide; glimepiride and gliclazide. Tolbutamide, glibenclamide, gliclazide, glibornuride, gliquidone, glisoxepid and glimepiride can be administered in the form as they are marketed under the trademarks RASTINON HOECHST™, AZUGLUCON™, DIAMICRONT™, GLUBORID™, GLURENORM™, PRO-DIABAN™ and AMARYL™, respectively.

In another variation, such combined, single dose form comprises Compound I and an antidiabetic D-phenylalanine derivative. Particular examples of antidiabetic D-phenylalanine derivatives that may be used in this variation include, but are not limited to repaglinide and nateglinide which may be administered in the form as they are marketed under the trademarks NOVONORM™ and STARLIX™, respectively.

In another variation, such combined, single dose form comprises Compound I and an alpha-Glucosidase inhibitor. Particular examples of alpha-Glucosidase inhibitors that may be used in this variation include, but are not limited to acarbose, miglitol and voglibose which may be administered in the form as they are marketed under the trademarks GLUCOBAY™, DIASTABOL 50™ and BASEN™, respectively.

In one particular embodiment, the antidiabetic compound administered in combination with Compound I in such combined, single dose form is selected from the group consisting of nateglinide, repaglinide, metformin, extendatide, rosiglitazone, pioglitazone, glisoxepid, glyburide, glibenclamide, acetohexamide, chloropropamide, glibornuride, tolbutamide, tolazamide, glipizide, carbutamide, gliquidone, glyhexamide, phenbutamide, tolcyclamide, glimepiride and gliclazide, including any pharmaceutically acceptable salts thereof.

In regard to each of the above embodiments and variations regarding a combined, single dose form comprising the combination of Compound I and one or more other antidiabetic compounds, the pharmaceutical composition may optionally be adapted for oral administration and in this regard may optionally be a solid formulation such as a tablet or capsule or may alternatively be in a liquid formulation adapted for oral administration. The dose of the antidiabetic compound may be selected from the range known to be clinically employed for such compound. Any of therapeutic compounds of diabetic complications, antihyperlipemic compounds, antiobestic compounds or antihypertensive compounds can be used in combination with Compound I in the same manner as the above antidiabetic compounds. Examples of therapeutic compounds of diabetic complications include, but are not limited to, aldose reductase inhibitors such as tolrestat, epalrestat, zenarestat, zopolrestat, minalrestat, fidarestat, CT-112 and ranirestat; neurotrophic factors and increasing compounds thereof such as NGF, NT-3, BDNF and neurotrophin production-secretion promoters described in WO01/14372 (e.g., 4-(4-chlorophenyl)-2-(2-methyl-1-imidazolyl)-5-[3-(2-methylphenoxy)propyl]oxazole); neuranagenesis stimulators such as Y-128; PKC inhibitors such as ruboxistaurin mesylate; AGE inhibitors such as ALT946, pimagedine, N-phenacylthiazolium bromide (ALT766), ALT-711, EXO-226, pyridorin and pyridoxamine; reactive oxygen scavengers such as thioctic acid; cerebral vasodilators such as tiapride and mexiletine; somatostatin receptor agonists such as BIM23190; and apoptosis signal regulating kinase-1 (ASK-1) inhibitors. Examples of antihyperlipemic compounds include, but are not limited to, HMG-CoA reductase inhibitors such as pravastatin, simvastatin, lovastatin, atorvastatin, fluvastatin, rosuvastatin and pitavastatin; squalene synthase inhibitors such as compounds described in WO97/10224 (e.g., N-[[(3R,55)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid); fibrate compounds such as bezafibrate, clofibrate, simfibrate and clinofibrate; ACAT inhibitors such as avasimibe and eflucimibe; anion exchange resins such as colestyramine; probucol; nicotinic acid drugs such as nicomol and niceritrol; ethyl icosapentate; and plant sterols such as soysterol and γ-oryzanol. Examples of antiobestic compounds include, but are not limited to, dexfenfluramine, fenfluramine, phentermine, sibutramine, amfepramone, dexamphetamine, mazindol, phenylpropanolamine, clobenzorex; MCH receptor antagonists such as SB-568849 and SNAP-7941; neuropeptide Y antagonists such as CP-422935; cannabinoid receptor antagonists such as SR-141716 and SR-147778; ghrelin antagonist; 11β-hydroxysteroid dehydrogenase inhibitors such as BVT-3498; pancreatic lipase inhibitors such as orlistat and ATL-962; Beta-3 AR agonists such as AJ-9677; peptidic anorexiants such as leptin and CNTF (Ciliary Neurotropic Factor); cholecystokinin agonists such as lintitript and FPL-15849; and feeding deterrent such as P-57. Examples of the antihypertensive compounds include angiotensin converting enzyme inhibitors such as captopril, enalapril and delapril; angiotensin II antagonists such as candesartan cilexetil, losartan, eprosartan, valsartan, telmisartan, irbesartan, olmesartan medoxomil, tasosartan and 1-[[2'-(2,5-dihydro-5-oxo-4H-1,2,4-oxadiazol-3-yl)biphenyl-4-yl]methyl]-2-ethoxy-1H-benzimidazole-7-carboxylic acid; calcium channel blockers such as manidipine, nifedipine, nicardipine, amlodipine and efonidipine; potassium channel openers such as levcromakalim, L-27152, AL0671 and NIP-121; and clonidine.

### 5. KITS AND ARTICLES OF MANUFACTURE COMPRISING COMPOUND I

The present invention also relates to kits comprising a pharmaceutical composition according to the present invention comprising Compound 1 (and optionally one or more other antidiabetic compounds) where such kit further comprises instructions that include one or more forms of information selected from the group consisting of indicating a disease state for which the pharmaceutical composition is to be administered, storage information for the pharmaceutical composition, dosing information and instructions regarding how to administer the pharmaceutical composition. The kit may also comprise packaging materials. The packaging material may also comprise a container for housing the pharmaceutical composition. The container may optionally comprise a label indicating the disease state for which the pharmaceutical composition is to be administered, storage information, dosing information and/or instructions regarding how to administer the composition. The kit may also comprise additional components for storage or administration of the composition. The kit may also comprise the composition in single or multiple dose forms.

In one embodiment, the pharmaceutical composition in the kit comprises multiple doses of a pharmaceutical composition according to the present invention wherein such pharmaceutical composition is a single dose form that comprises Compound I in one of the dosage ranges specified herein.

In another embodiment, the pharmaceutical composition in the kit comprises multiple doses of a pharmaceutical composition according to the present invention wherein such pharmaceutical composition is a single dose form that comprises Compound I and one or more of the other antidiabetic compounds specified herein.

The present invention also relates to articles of manufacture comprising a pharmaceutical composition according to the present invention comprising Compound I (and optionally one or more other antidiabetic compounds) where such articles of manufacture further comprise packaging materials. In one variation, the packaging material comprises a container for housing the composition. In another variation, the invention provides an article of manufacture where the container comprises a label indicating one or more members of the group consisting of a disease state for which the composition is to be administered, storage information, dosing information and/or instructions regarding how to administer the composition.

In one embodiment, the pharmaceutical composition in the article of manufacture comprises multiple doses of a pharmaceutical composition according to the present invention wherein such pharmaceutical composition is a single dose form that comprises Compound I in one of the dosage ranges specified herein.

In another embodiment, the pharmaceutical composition in the article of manufacture comprises multiple doses of a pharmaceutical composition according to the present invention wherein such pharmaceutical composition is a single dose form that comprises Compound I and one or more of the other antidiabetic compounds specified herein.

It is noted that the packaging material used in kits and articles of manufacture according to the present invention may form a plurality of divided containers such as a divided bottle or a divided foil packet. The container can be in any conventional shape or form as known in the art which is made of a pharmaceutically acceptable material, for example a paper or cardboard box, a glass or plastic bottle or jar, a re-sealable bag (for example, to hold a "refill" of tablets for placement into a different container), or a blister pack with individual doses for pressing out of the pack according to a therapeutic schedule. The container that is employed will depend on the exact dosage form involved. It is feasible that more than one container can be used together in a single package to market a single dosage form. For example, tablets may be contained in a bottle that is in turn contained within a box.

One particular example of a kit according to the present invention is a so-called blister pack. Blister packs are well known in the packaging industry and are being widely used for the packaging of pharmaceutical unit dosage forms (tablets, capsules, and the like). Blister packs generally consist of a sheet of relatively stiff material (preferably stiff transparent plastic material) covered with a foil. During the packaging process recesses are formed in the stiff material. The recesses have the size and shape of individual tablets or capsules to be packed or may have the size and shape to accommodate multiple tablets and/or capsules to be packed. Next, the tablets or capsules are placed in the recesses accordingly and the sheet of relatively stiff material is sealed against the plastic foil at the face of the foil which is opposite from the direction in which the recesses were formed. As a result, the tablets or capsules arc individually scaled or collectively sealed, as desired, in the recesses between the foil and the sheet. The strength of the sheet is preferably such that the tablets or capsules can be removed from the blister pack by manually applying pressure on the recesses whereby an opening is formed in the foil at the place of the recess. The tablet or capsule can then be removed via said opening.

### EXAMPLES

### 1. Preparation of 2-[6-(3-Amino-piperidin-1-yl)-3-methyl-2,4-dioxo-3,4-dihydro-2H-pyrimidin-1-ylmethyl]-4-fluoro-benzonitrile and pharmaceutically acceptable salts

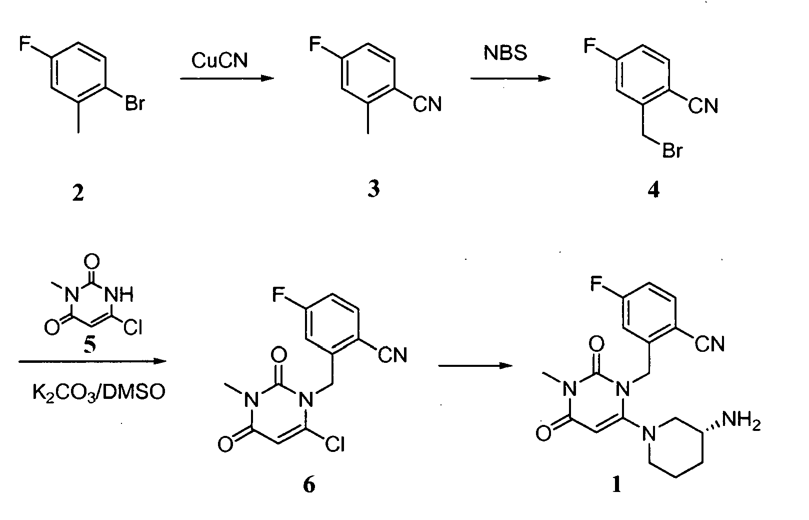

### 4-Fluoro-2-methylbenzonitrile (3)

A mixture of 2-bromo-5fluorotoluene ( 2) (3.5 g, 18.5 mmol) and CuCN (2 g, 22 mmol) in DMF (100 mL) was refluxed for 24 hours. The reaction was diluted with water and extracted with hexane. The organics were dried over MgSO₄ and the solvent removed to give product 3 (yield 60%). ¹H-NMR (400 MHz, CDCl₃): δ 7.60 (dd, J=5.6, 8.8 Hz, 1H), 6.93-7.06 (m, 2H), 2.55 (s, 3H).

### 2-Bromomethyl-4-fluorobenzonitrile (4)

A mixture of 4-fluoro-2-methylbenzonitrile (3) (2 g, 14.8 mmol), NBS (2.64 g, 15 mmol) and AlBN (100 mg) in CCl₄ was refluxed under nitrogen for 2 hours. The reaction was cooled to room temperature. The solid was removed by filtration. The organic solution was concentrated to give crude product as an oil, which was used in the next step without further purification. ¹H-NMR (400 MHz, CDCl₃): δ 7.68 (dd, J= 5.2, 8.4 Hz, 1H), 7.28 (dd, J= 2.4, 8.8 Hz, 1H), 7.12 (m, 1H), 4.6 (s, 2H).

### 2-(6-Chloro-3-methyl-2,4-dioxo-3,4-dihydro-2H-pyrimidin-1-ylmethyl)-4-fluoro-benzonitrile (6)

A mixture of crude 3-methyl-6-chlorouracil (5) (0.6 g, 3.8 mmol), 2-Bromomethyl-4-fluorobenzonitrile (0.86 g, 4 mmol) and K₂CO₃ (0.5 g, 4 mmol) in DMSO (10 mL) was stirred at 60 °C for 2 hours. The reaction was diluted with water and extracted with EtOAc. The organics were dried over MgSO₄ and the solvent removed. The residue was purified by column chromatography. 0.66 g of the product was obtained (yield: 60%). ¹H-NMR (400 MHz, CDCl₃): δ 7.73 (dd, J=7.2, 8.4Hz, 1H), 7.26 (d, J-4.0Hz, 1H), 7.11-7.17 (m, 1H), 6.94 (dd, J=2.0, 9.0 Hz, 1H), 6.034 (s, 2H), 3.39 (s, 3H). MS (ES) [m+H] calc'd for C₁₃H₉ClFN₃O₂, 293.68; found 293.68.

### 2-[6-(3-Amino-piperidin-1-yl)-3-methyl-2,4-dioxo-3,4-dihydro-2H-pyrimidin-1-ylmethyl]-4-fluoro-benzonitrile, TFA salt (1) (TFA salt of Compound I)

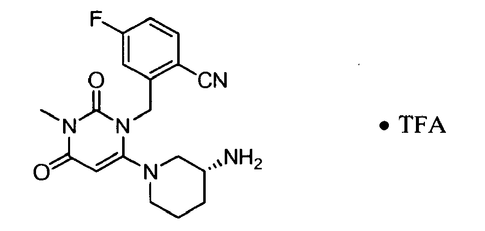

2-(6-Chloro-3-methyl-2,4-dioxo-3,4-dihydro-2H-pyrimidin-1-ylmethyl)-4-fluoro-benzonitrile (5) (300 mg, 1.0 mmol), (R)-3-amino-piperidine dihydrochloride (266 mg, 1.5 mmol) and sodium bicarbonate (500 mg, 5.4 mmol) were stirred in a sealed tube in EtOH (3 mL) at 100 °C for 2 hrs. The final compound was obtained as a TFA salt after HPLC purification. ¹H-NMR (400 MHz, CD₃OD): δ. 7.77-7.84 (m, 1H), 7.16-7.27 (m, 2H), 5.46 (s, 1H), 5.17-5.34 (ABq, 2H, J = 35.2, 15.6 Hz), 3.33-3.47 (m, 2H), 3.22 (s, 3H), 2.98-3.08 (m, 1H), 2.67-2.92 (m, 2H), 2.07-2.17 (m, 1H), 1.82-1.92 (m, 1H), 1.51-1.79 (m, 2H). MS (ES) [m+H] calc'd for C₁₈H₂₀FN₅O₂, 357.38; found, 357.38.

### 2-[6-(3-Amino-piperidin-1-yl)-3-methyl-2,4-dioxo-3,4-dihydro-2H-pyrimidin-1-ylmethyl]-4-fluoro-benzonitrile, HCl salt

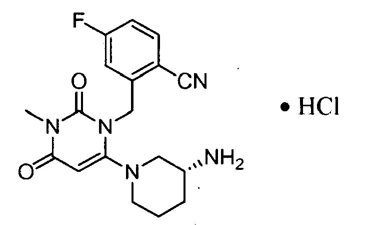

The TFA salt of Compound I was suspended in DCM, and then washed with saturated Na₂CO₃. The organic layer was dried and removed in vacuo. The residue was dissolved in acetonitrile and HCl in dioxane (1.5 eq.) was added at 0 °C. The HCl salt was obtained after removing the solvent. ¹H-NMR (400 MHz, CD₃OD): δ. 7.77-7.84 (m, 1H), 7.12-7.26 (m, 2H), 5.47 (s, 1H), 5.21-5.32 (ABq, 2H, J = 32.0, 16.0 Hz), 3.35-3.5 (m, 2H), 3.22 (s, 3H), 3.01-3.1 (m, 1H), 2.69-2.93 (m, 2H), 2.07-2.17 (m, 1H), 1.83-1.93 (m, 1H), 1.55-1.80 (m, 2H). MS (ES) [m+H] calc'd for C₁₈H₂₀FN₅O₂, 357.38; found, 357.38.

### General procedure for the preparation of salts of Compound I.

The benzonitrile product may be isolated as the free base if desired, but preferably, the product may be further converted to a corresponding acid addition salt. Specifically, the benzonitrile product (approximately 10 mg) in a solution of MeOH (1 mL) was treated with various acids (1.05 equivalents). The solutions were allowed to stand for three days open to the air. If a precipitate formed, the mixture was filtered and the salt dried. If no solid formed, the mixture was concentrated in vacuo and the residue isolated. In this way, salts of Compound I were prepared from the following acids: benzoic, p-toluenesulfonic, succinic, R-(-)-Mandelic and benzenesulfonic.

The isolation and/or purification steps of the intermediate compounds in the above described process may optionally be avoided if the intermediates from the reaction mixture are obtained as relatively pure compounds and the by-products or impurities of the reaction mixture do not interfere with the subsequent reaction steps. Where feasible, one or more isolation steps may be eliminated to provide shorter processing times, and the elimination of further processing may also afford higher overall reaction yields.

### 2. Exemplary formulations comprising succinate salt of 2-[6-(3-Amino-piperidin-1-yl)-3-methyl-1-2,4-dioxo-3,4-dihydro-2H-pyrimidin-1-ylmethyl]-4-fluoro-benzonitrile

Provided are examples of tablet formulations that may be used to administer succinate salt of 2-[6-(3-Amino-piperidin-1-yl)-3-methyl-2,4-dioxo-3,4-dihydro-2H-pyrimidin-1-ylmethyl]-4-fluoro-benzonitrile (Succinate salt of Compound I) according to the present invention. It is noted that the formulations provided herein may be varied as is known in the art.

The exemplary tablet formulations are as follows:

**12.5mg of Compound I (weight of free base form) per tablet**

| Core Tablet Formulation | | |
|---|---|---|
| (1) | 2-[6-(3-Amino-piperidin-1-yl)-3-methyl-2,4-dioxo-3,4-dihydro-2H-pyrimidin-1-ylmethyl]-4-fluoro-benzonitrile (succinate salt) | 17.0 mg |
| (2) | Lactose Monohydrate, NF, Ph, Eur (FOREMOST 316 FAST FLO) | 224.6 mg |
| (3) | Microcrystalline Cellulose, NF, Ph, Eur (AVICEL PH 102) | 120.1 mg |
| (4) | Croscarmellose Sodium, NF, Ph, Eur (AC-DO-SOL) | 32.0 mg |
| (5) | Colloidal Silicon Dioxide, NF, Ph, Eur (CAB-O-SIL M-5P) | 3.2 mg |
| (6) | Magnesium Stearate, NF, Ph, Eur (MALLINCKRODT, Non-bovine Hyqual) | 3.2 mg |
| | TOTAL (per tablet) | 400.0 mg |
| | | |

| Film Coat (12.0 mg in total) | | |
|---|---|---|
| (1) | Opadry II 85F18422, White - Portion 1 (COLORCON) | |
| (2) | Opadry II 85F18422, White - Portion 2 (COLORCON) | |
| (3) | Opadry II 85F18422, White - Portion 3 (COLORCON) | |

**25mg of Compound I (weight of free base form) per tablet**

| Core Tablet Formulation | | |
|---|---|---|
| (1) | 2-[6-(3-Amino-piperidin-1-yl)-3-methyl-2,4-dioxo-3,4-dihydro-2H-pyrimidin-1-ylmethyl]-4-fluoro-benzonitrile (succinate salt) | 34.0 mg |
| (2) | Lactose Monohydrate, NF, Ph, Eur (FOREMOST 316 FAST FLO) | 207.6 mg |
| (3) | Microcrystalline Cellulose, NF, Ph, Eur (AVICEL PH 102) | 120.1 mg |
| (4) | Croscarmellose Sodium, NF, Ph, Eur (AC-DO-SOL) | 32.0 mg |
| (5) | Colloidal Silicon Dioxide, NF, Ph, Eur (CAB-O-SIL M-5P) | 3.2 mg |
| (6) | Magnesium Stearate, NF, Ph, Eur (MALLINCKRODT, Non-bovine Hyqual) | 3.2 mg |
| | TOTAL (per tablet) | 400.0 mg |
| | | |

| Film Coat (12.0 mg in total) | | |
|---|---|---|
| (1) | Opadry II 85F 18422, White - Portion 1 (COLORCON) | |
| (2) | Opadry II 85F 18422, White - Portion 2 (COLORCON) | |
| (3) | Opadry II 85F18422, White - Portion 3 (COLORCON) | |

**50mg of Compound I (weight of free base form) per tablet**

| Core Tablet Formulation | | |
|---|---|---|
| (1) | 2-[6-(3-Amino-piperidin-1-yl)-3-methyl-2,4-dioxo-3,4-dihydro-2H-pyrimidin-1-ylmethyl]-4-fluoro-benzonitrile (succinate salt) | 68.0 mg |
| (2) | Lactose Monohydrate, NF, Ph, Eur (FOREMOST 316 FAST FLO) | 173.6 mg |
| (3) | Microcrystalline Cellulose, NF, Ph, Eur (AVICEL PH 102) | 120.1 mg |
| (4) | Croscarmellose Sodium, NF, Ph, Eur (AC-DO-SOL) | 32.0 mg |
| (5) | Colloidal Silicon Dioxide, NF, Ph, Eur (CAB-O-SIL M-5P) | 3.2 mg |
| (6) | Magnesium Stearate, NF, Ph, Eur (MALLINCKRODT, Non-bovine Hyqual) | 3.2 mg |
| | TOTAL (per tablet) | 400.0 mg |
| | | |

| Film Coat (12.0 mg in total) | | |
|---|---|---|
| (1) | Opadry II 85F 18422, White - Portion I (COLORCON) | |
| (2) | Opadry II 85F18422, White - Portion 2 (COLORCON) | |
| (3) | Opadry II 85F18422, White - Portion 3 (COLORCON) | |

### 6. EFFECT OF ADMINISTRATION ON PLASMA DPP-IV ACTIVITY

A single dose of Compound I was administered orally to monkey at a dosage of 0.3 mg/kg. Figure 1 illustrates (for reference purposes) the observed effect that administering Compound I has on the monkey's plasma DPPIV activity post dosing. As can be seen, Compound I reduced DPP-IV activity in monkey's plasma by greater than 90% relative to baseline at 12 hours post dosing. In view of the data presented, it is believed that when at least 25mg of Compound I is administered to a patient, the patient's plasma DPPIV activity may be reduced by greater than 60% relative to baseline for a period of at least at least 6 hours, 12 hours, 18 hours and even 24 hours following administration.

### 7. EFFECT OF CO-ADMINISTRATION WITH PIOGLITAZONE ON PLASMA GLUCOSE

The effect of administering Compound I in combination with pioglitazone was investigated by measuring plasma glucose levels in mice. Male *db*/*db* (BKS.Cg-*+Lepr^{db}*/+*Lepr^{db}*) mice (6 weeks of age, CLEA Japan (Tokyo, Japan)) were divided into 4 groups (n=7 in each group) comprising Group A to Group D. Group A had free access to CE-2 powder chow (CLEA Japan) for 21 days. Group B had free access to CE-2 powder chow (CLEA Japan) containing 0.03% (w/w) of succinate salt of Compound I for 21 days. The dose of Compound I in Group B was calculated to be 74.8±2.5 (mean ± SD) mg/kg body weight/day. Group C had free access to CE-2 powder chow (CLEA Japan) containing 0.0075%(w/w) of pioglitazone hydrochloride for 21 days. The dose of pioglitazone in Group C was calculated to be 17.7±0.6 (mean ± SD) mg/kg body weight/day. Group D had free access to CE-2 powder chow (CLEA Japan) containing 0.03% (w/w) of succinate salt of Compound I in combination with 0.0075% (w/w) of pioglitazone hydrochloride for 21 days. The doses of Compound I and pioglitazone in Group D were calculated to be 63.1±1.9 (mean ± SD) mg/kg body weight/day and 15.8±0.5 (mean ± SD) mg/kg body weight/day, respectively. During 21 days of administration of the powder chow, there were not significant differences in the administration amount of the powder chow in the above 4 groups. After 21 days of administration of the powder chow, blood samples were taken from the orbital veins of the mice by capillary pipette under feeding condition, and plasma glucose levels were enzymatically measured by using Autoanalyzer 7080 (Hitachi, Japan).

The results are shown in Table 1. The values in the table means average (n=7) ± standard deviation.

**Table 1**

| **Group** | **Plasma Glucose** **(mg/dL)** |
|---|---|
| Group A (control) | 472.9 ± 74.6 |
| Group B (Compound I) | 410.3 ± 47.9 |
| Group C (Pioglitazone) | 394.4 ± 47.9 |
| Group D (Compound I + Pioglitazone) | 256.1 ± 62.5 |

As shown in Table 1, the combination of Compound I with pioglitazone showed excellent effects of lowering plasma glucose levels.

### 8. EFFECT OF ADMINISTRATION ON PLASMA DPP-IV ACTIVITY

A single dose of Compound I was administered orally to 6 humans at a dosage of 12.5 mg, 25 mg, 50 mg, 100 mg, 200 mg and 400 mg, respectively (total 36 humans). Figure 2 illustrates the observed effect that administering Compound I has on the human plasma DPP-IV activity post dosing. As can be seen, Compound I reduced DPP-IV activity in human plasma by greater than 10% relative to baseline at 168 hours post dosing. Thus, as can be seen from the data shown in Figure 2, by administering Compound I one time per week at the dosage levels specified herein, Compound I can be effectively used relative to disease states where it is desired to reduce plasma DPP-IV activity. In view of the data presented, it is believed that when at least 50 mg of Compound I is administered to a patient, the patient's plasma DPP-IV activity may be reduced by greater than 35% relative to baseline for a period of at least 168 hours following administration, when at least 100 mg of Compound I is administered to a patient, the patient's plasma DPP-IV activity may be reduced by greater than 60% relative to baseline for a period of at least 168 hours following administration, and , when at least 200 mg of Compound I is administered to a patient, the patient's plasma DPP-IV activity may be reduced by greater than 70% relative to baseline for a period of at least 168 hours following administration.

### 9. EFFECT OF ADMINISTRATION ON PLASMA DPP-IV ACTIVITY

A single dose of Compound I was administered orally to humans at a dosage of 3.125 mg (to 9 humans), 12.5 mg (to 8 humans), 50 mg (to 7 humans) and 100 mg (to 8 humans), respectively. Figure 3 illustrates the observed effect that administering Compound I has on the human plasma DPP-IV activity post dosing. As can be seen, Compound I reduced DPP-IV activity in human plasma by greater than 20% relative to baseline at 168 hours post dosing. Thus, as can be seen from the data shown in Figure 3, by administering Compound I one time per week at the dosage levels specified herein, Compound I can be effectively used relative to disease states where it is desired to reduce plasma DPP-IV activity. In view of the data presented, it is believed that when at least 50 mg of Compound I is administered to a patient, the patient's plasma DPP-IV activity may be reduced by greater than 65% relative to baseline for a period of at least 168 hours following administration.

It will be apparent to those skilled in the art that various modifications and variations can be made to the compounds, compositions, kits, and methods of the present invention without departing from the scope of the invention. Thus, it is intended that the present invention cover the modifications and variations of this invention provided they come within the scope of the appended claims.

## Claims

1. 2-[6-(3-Amino-piperidin-1-yl)-3-methyl-2,4-dioxo-3,4-dihydro-2H-pyrimidin-1-ylmethyl]-4-fluoro-benzonitrile (Compound I) for use as a medicament when administered at a weekly dose of between 50 mg and 250 mg, wherein the weekly dose is administered once per week.

2. The compound according to claim 1, wherein the weekly dose of Compound I administered to the patient is between 100 mg and 250 mg.

3. The compound according to claim 1, wherein the weekly dose of Compound I administered to the patient is 50 mg, 75 mg, 100 mg, 125 mg, 150 mg, 175 mg or 200 mg.

4. 2-[6-(3-Amino-piperidin-1-yl)-3-methyl-2,4-dioxo-3,4-dihydro-2H-pyrimidin-1-ylmethyl]-4-fluoro-benzonitrile (Compound I) for use in treating diabetes when administered at a weekly dose of between 50 mg and 250 mg, wherein the weekly dose is administered once per week.

5. 2-[6-(3-Amino-piperidin-1-yl)-3-methyl-2,4-dioxo-3,4-dihydro-2H-pyrimidin-1-ylmethyl]-4-fluoro-benzonitrile (Compound I) for use in treating cancer when administered at a weekly dose of between 50 mg and 250 mg, wherein the weekly dose is administered once per week.

6. 2-[6-(3-Amino-piperidin-1-yl)-3-methyl-2,4-dioxo-3,4-dihydro-2H-pyrimidin-1-ylmethyl]-4-fluoro-benzonitrile (Compound I) for use in treating autoimmune disorders when administered at a weekly dose of between 50 mg and 250 mg, wherein the weekly dose is administered once per week.

7. 2-[6-(3-Amino-piperidin-1-yl)-3-methyl-2,4-dioxo-3,4-dihydro-2H-pyrimidin-1-ylmethyl]-4-fluoro-benzonitrile (Compound I) for use in treating HIV infection when administered at a weekly dose of between 50 mg and 250 mg, wherein the weekly dose is administered once per week.

8. Use of 2-[6-(3-Amino-piperidin-1-yl)-3-methyl-2,4-dioxo-3,4-dihydro-2H-pyrimidin-1-ylmethyl]-4-fluoro-benzonitrile (Compound I) in the manufacture of a medicament for treating a disease or condition selected from diabetes, cancer, an autoimmune disorder or HIV infection, wherein the medicament is formulated such that Compound I is administered at a weekly dose of between 50 mg and 250 mg, wherein the weekly dose is administered once per week.

## Patentansprüche

1. 2-[6-(3-Amino-piperidin-1-yl)-3-methyl-2,4-dioxo-3,4-dihydro-2H-pyrimidin-1-ylmethyl]-4-fluor-benzonitril (Verbindung I) für die Verwendung als Medikament, wenn es in einer wöchentlichen Dosis verabreicht wird, die zwischen 50 mg und 250 mg liegt, wobei die wöchentliche Dosis einmal pro Woche verabreicht wird.

2. Verbindung gemäß Anspruch 1, wobei die wöchentliche Dosis der Verbindung I, die dem Patienten verabreicht wird, zwischen 100 mg und 250 mg liegt.

3. Verbindung gemäß Anspruch 1, wobei die wöchentliche Dosis der Verbindung I, die dem Patienten verabreicht wird, 50 mg, 75 mg, 100 mg, 125 mg, 150 mg, 175 mg oder 200 mg ist.

4. 2-[6-(3-Amino-piperidin-1-yl)-3-methyl-2,4-dioxo-3,4-dihydro-2H-pyrimidin-1-ylmethyl]-4-fluor-benzonitril (Verbindung I) für die Verwerdung bei der Behandlung von Diabetes, wenn es in einer wöchentlichen Dosis verabreicht wird, die zwischen 50 mg und 250 mg liegt, wobei die wöchentliche Dosis einmal pro Woche verabreicht wird.

5. 2-[6-(3-Amino-piperidin-1-yl)-3-methyl-2,4-dioxo-3,4-dihydro-2H-pyrimidin-1-ylmethyl]-4-fluor-benzonitril (Verbindung I) für die Verwendung bei der Behandlung von Krebs, wenn es in einer wöchentlichen Dosis verabreicht wird, die zwischen 50 mg und 250 mg liegt, wobei die wöchentliche Dosis einmal pro Woche verabreicht wird.

6. 2-[6-(3-Amino-piperidin-1-yl)-3-methyl-2,4-dioxo-3,4-dihydro-2H-pyrimidin-1-ylmethyl]-4-fluor-benzonitril (Verbindung I) für die Verwerdung bei der Behandlung von Autoimmunkrankheiten, wenn es in einer wöchentlichen Dosis verabreicht wird, die zwischen 50 mg und 250 mg liegt, wobei die wöchentliche Dosis einmal pro Woche verabreicht wird.

7. 2-[6-(3-Amino-piperidin-1-yl)-3-methyl-2,4-dioxo-3,4-dihydro-2H-pyrimidin-1-ylmethyl]-4-fluor-benzonitril (Verbindung I) für die Verwendung bei der Behandlung einer HIV-Infektion, wenn es in einer wöchentlichen Dosis verabreicht wird, die zwischen 50 mg und 250 mg liegt, wobei die wöchentliche Dosis einmal pro Woche verabreicht wird.

8. Verwendung von 2-[6-(3-Amino-piperidin-1-yl)-3-methyl-2,4-dioxo-3,4-dihydro-2H-pyrimidin-1-ylmethyl]-4-fluor-benzonitril (Verbindung I) bei der Herstellung eines Medikaments zur Behandlung einer Krankheit oder eines Zustands, ausgewählt aus Diabetes, Krebs, einer Autoimmunkrankheit oder einer HIV-Infektion, wobei das Medikament so formuliert ist, dass die Verbindung I in einer wöchentlichen Dosis verabreicht wird, die zwischen 50 mg und 250 mg liegt, wobei die wöchentliche Dosis einmal pro Woche verabreicht wird.

## Revendications

1. 2-[6-(3-amino-pipéridin-1-yl)-3-méthyl-2,4-dioxo-3,4-dihydro-2H-pyrimidin-1-ylméthyl]-4-fluoro-benzonitrile (composé I) destiné à être utilisé comme médicament quand il est administré à une dose hebdomadaire entre 50 mg et 250 mg, où la dose hebdomadaire est administrée une fois par semaine.

2. Composé selon la revendication 1 où la dose hebdomadaire de composé I administrée au patient est entre 100 mg et 250 mg.

3. Composé selon la revendication 1 où la dose hebdomadaire de composé I administrée au patient est 50 mg, 75 mg, 100 mg, 125 mg, 150 mg, 175 mg ou 200 mg.

4. 2-[6-(3-amino-pipéridin-1-yl)-3-méthyl-2,4-dioxo-3,4-dihydro-2H-pyrimidin-1-ylméthyl]-4-fluoro-benzonitrile (composé I) destiné à être utilisé dans le traitement du diabète quand il est administré à une dose hebdomadaire entre 50 mg et 250 mg, où la dose hebdomadaire est administrée une fois par semaine.

5. 2-[6-(3-amino-pipéridin-1-yl)-3-méthyl-2,4-dioxo-3,4-dihydro-2H-pyrimidin-1-ylméthyl]-4-fluoro-benzonitrile (composé I) destiné à être utilisé dans le traitement du cancer quand il est administré à une dose hebdomadaire entre 50 mg et 250 mg, où la dose hebdomadaire est administrée une fois par semaine.

6. 2-[6-(3-amino-pipéridin-1-yl)-3-méthyl-2,4-dioxo-3,4-dihydro-2H-pyrimidin-1-ylméthyl]-4-fluoro-benzonitrile (composé I) destiné à être utilisé dans le traitement des troubles auto-immuns quand il est administré à une dose hebdomadaire entre 50 mg et 250 mg, où la dose hebdomadaire est administrée une fois par semaine.

7. 2-[6-(3-amino-pipéridin-1-yl)-3-méthyl-2,4-dioxo-3,4-dihydro-2H-pyrimidin-1-ylméthyl]-4-fluoro-benzonitrile (composé I) destiné à être utilisé dans le traitement d'une infection à VIH quand il est administré à une dose hebdomadaire entre 50 mg et 250 mg, où la dose hebdomadaire est administrée une fois par semaine.

8. Utilisation du 2-[6-(3-amino-pipéridin-1-yl)-3-méthyl-2,4-dioxo-3,4-dihydro-2H-pyrimidin-1-ylméthyl]-4-fluoro-benzonitrile (composé I) dans la fabrication d'un médicament pour traiter une maladie ou affection choisie parmi le diabète, le cancer, un trouble auto-immun et une infection à VIH, où le médicament est formulé de telle sorte que le composé 1 est administré à une dose hebdomadaire entre 50 mg et 250 mg, où la dose hebdomadaire est administrée une fois par semaine.
